# EUROPEAN PATENT APPLICATION

(11) **EP 1 900 302 A1**
(43) Date of publication of application: **19.03.2008**
(21) Application number: 06019210.1
(22) Date of filing: 13.09.2006
(51) Int. Cl.: A46B 5/04, A61Q 11/00

(54) **Tooth cleaner**

(71) Applicant: Adwin Korea Corporation, Kangnam-ku, Seoul (KR)
(72) Inventor: Park, Han-Wook, Songpa-gu Seoul (KR)
(74) Representative: Casalonga, Axel

(57) **Abstract**

The present invention relates to a tooth cleaner that is put on a finger and covered with a mouth detergent on one side thereof so as to cleanse teeth, and relates to a tooth cleaner for achieving good tooth cleansing performance and having a configuration that may be mass produced. The tooth cleaner includes a substrate, a sheet attached to one surface of the substrate to form a finger cover, and a tooth cleansing portion attached to another surface of the substrate and having a mouth detergent. The tooth cleansing portion may be formed by one of a paste having the mouth detergent and covering the other surface of the substrate, cotton or a sponge having the mouth detergent, two embossed paper napkins that are symmetrically superposed wherein a space formed between the two embossed paper napkins is charged with the mouth detergent, a water expansile material having the mouth detergent, a porous non-woven fabric charged with the mouth detergent, soft silicon dots arranged in a predetermined pattern wherein the mouth detergent is charged between the arranged soft silicon dots, or a liquid mouth detergent layer and a solid mouth detergent layer wherein liquid and solid layers are stacked on the other surface of the substrate.

## Description

The present invention relates to a tooth cleaner, and it more particularly relates to a tooth cleaner for rubbing teeth and gingivae by using a soft tooth cleansing portion so that the teeth may be cleansed and massaged, and formed by a simple configuration so that it is handy to carry and is easy to use while traveling or being involved in an activity.

The present assignee has suggested a tooth cleaner for cleansing teeth without using a tooth brush and tooth paste while traveling or being involved in an activity. Since the tooth cleaner is handy to carry, it is easy to maintain cleanliness of teeth without the inconvenience of using tooth paste.

Since a fabric panel is cut to a predetermined size and folded several times to integrally form a finger covering portion and a tooth cleansing portion having a mouth detergent, the resultant tooth cleaner involves much manual work, and it is not appropriate for mass production.

Differing from the above tooth cleaner, a narrow cotton tissue corresponding to the size of the finger covering portion is put on an embossed surface of non-woven fabric having the mouth detergent, the narrow cotton tissue and the embossed surface are pressed down by a mold to perform ultrasonic bonding and to form a shape, and therefore, mass production of the tooth cleaner may be achieved.

However, since teeth are rubbed and cleansed by the embossed surface of the non-woven fabric, the mass produced tooth cleaner has the following problems.

Firstly, since the non-woven fabric of the tooth cleaner is formed from inferior chemical textiles, shag is raised on the embossed surface when the teeth are rubbed by the embossed surface.

Secondly, since it is difficult to cleanse gaps between the teeth by the embossed surface of the non-woven fabric, a cleansing effect and a user's feeling of cleanness are low.

The above information disclosed in this Background section is only for enhancement of understanding of the background of the invention and therefore it may contain information that does not form the prior art that is already known in this country to a person of ordinary skill in the art.

The present invention has been made in an effort to provide a tooth cleaner having a good tooth cleansing effect and an appropriate configuration for mass production. An exemplary tooth cleaner according to an embodiment of the present invention may massage and cleanse gaps between teeth, and it may be formed by bonding a finger covering portion to a substrate by an ultrasonic bonding operation.

The exemplary tooth cleaner includes a substrate, a sheet, and a tooth cleansing portion. The sheet is attached to one surface of the substrate to form a finger cover. The tooth cleansing portion having a mouth detergent is attached to another surface of the substrate.

The tooth cleansing portion may be formed by a water expansile material including the mouth detergent, a porous non-woven fabric charged with the mouth detergent, and cotton or a sponge having the mouth detergent, or by soft silicon dots arranged in a predetermined pattern and charged with the mouth detergent.

In addition, the tooth cleansing portion may include a liquid mouth detergent layer and a solid mouth detergent layer, and the liquid and solid mouth detergent layers are stacked on a surface of a substrate.

Further, the exemplary tooth cleaner may include a polishing layer provided on an outer surface of a sheet to eliminate tartar from teeth.

Still further, the exemplary tooth cleaner may include dental floss for cleansing a tongue and provided on the other surface of the substrate such that the dental floss is not overlapped with the tooth cleansing portion.
FIG. 1 shows a cross-sectional view of a tooth cleaner according to a first exemplary embodiment of the present invention.
FIG. 2 shows a cross-sectional view of a tooth cleaner according to a second exemplary embodiment of the present invention.
FIG. 3 shows a cross-sectional view of a tooth cleaner according to a third exemplary embodiment of the present invention.
FIG. 4 shows a cross-sectional view of a tooth cleaner according to a fourth exemplary embodiment according to the present invention.
FIG. 5 shows a cross-sectional view of a tooth cleaner according to a fifth exemplary embodiment of the present invention.
FIG. 6 shows a cross-sectional view of a tooth cleaner according to a sixth exemplary embodiment of the present invention.
FIG. 7 shows a cross-sectional view of a tooth cleaner according to a seventh exemplary embodiment of the present invention.
FIG. 8A and FIG. 8B respectively show manufacturing processes of the tooth cleaner according to the exemplary embodiment of the present invention.

### <Description of Reference Numerals Indicating Primary Elements in the Drawings>

| | | | |
|---|---|---|---|
| 2: | Substrate | 4: | Sheet |
| 6: | Finger covering portion | 8: | Tooth cleansing portion |
| 8a, 8b: | Paper napkins | | |
| 8c: | Mouth detergent | 8d: | Recess |
| 8e: | Liquid mouth detergent layer | 8f: | Solid mouth detergent layer |
| 10: | Protection film | 12: | Polishing layer |
| 14: | Dental floss | | |

An exemplary embodiment of the present invention will hereinafter be described in detail with reference to the accompanying drawings.

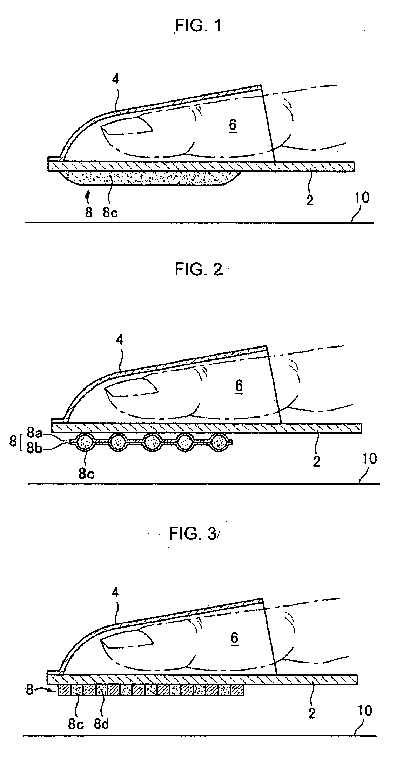

FIG. 1 shows a cross-sectional view of a tooth cleaner according to a first exemplary embodiment of the present invention. The tooth cleaner includes a substrate 2, a sheet 4 provided on one surface of the substrate 2 to form a finger covering portion 6, and a tooth cleansing portion 8 having a mouth detergent and provided on another surface of the substrate 2.

The substrate 2 is formed by a liquid absorbing fabric panel (e.g., the non-woven fabric, a fiber material, and a pulp material), or by adhering a polyethylene film on the non-woven fabric.

In the exemplary embodiment of the present invention, the tooth cleansing portion 8 is formed by a water expansile material so that the tooth cleansing portion 8 expands when it absorbs water. The water expansile material is formed by combining a highly absorbent pulp material and a resin, it remains with a minimum volume when it is dried, and it expands when absorbing water. The water expansile material is used for a water absorbing panel of an automatic desiccation cleaning device, which has been used as a substitution for a mop.

The tooth cleansing portion 8 formed by the water expansile material is put into a liquid mouth detergent so that the mouth detergent may be soaked into the tooth cleansing portion 8, and the tooth cleansing portion 8 is dried. In this case, ultrasonic bonding is performed to attach the tooth cleansing portion to the substrate 2.

In addition, among the tooth cleansing portion 8, a surface touching the teeth is formed in a shape with protrusions and depressions, and it is covered with a protection film 10 to protect the mouth detergent.

In this case, to use the tooth cleaner, a user takes off the protection film 10 from the tooth cleansing portion 8 and puts the finger covering portion 8 over a forefinger. The water expansile material forming the tooth cleansing portion 8 absorbs water and expands while brushing the teeth, so that the tooth cleansing effect is improved. In addition, since the mouth detergent is spilled out from the water expansile material while brushing the teeth, a tooth cleansing operation and a halitosis eliminating operation are performed.

FIG. 2 shows a cross-sectional view of a tooth cleaner according to a second exemplary embodiment of the present invention. A configuration of the tooth cleaner according to the second exemplary embodiment of the present invention is the same as that of the first exemplary embodiment of the present invention except that the tooth cleansing portion 8 includes two embossed paper napkins 8a and 8b that are symmetrically superposed, and a space formed between the two embossed paper napkins 8a and 8b is charged with a mouth detergent 8c.

According to the second exemplary embodiment of the present invention, gaps between the teeth are cleansed by an embossed surface of the paper napkin 8b of the tooth cleansing portion 8. In this case, since the embossed surface of the paper napkin 8b is symmetrically superposed, it has predetermined elasticity, and the tooth cleansing effect is further improved compared to a conventional embossed surface formed by one paper napkin.

FIG. 3 shows a cross-sectional view of a tooth cleaner according to a third exemplary embodiment of the present invention.

The tooth cleaner according to the third exemplary embodiment of the present invention is the same as that of the first exemplary embodiment of the present invention, except that the tooth cleansing portion 8 includes a porous non-woven fabric having a plurality of recesses 8d. The respective recesses 8d are charged with the mouth detergent 8c, and the protection film 10 covers the tooth cleansing portion 8.

Since the tooth cleansing portion 8 has the plurality of recesses 8d and a surface thereof is formed in a shape with protrusions and depressions, the tooth cleansing effect is improved. The mouth detergent charged in the respective recesses 8d is sufficient to brush the teeth.

According to the third exemplary embodiment of the present invention, the non-woven fabric forming the tooth cleansing portion 8 is integrally bonded to the substrate 2 by a thermal bonding method.

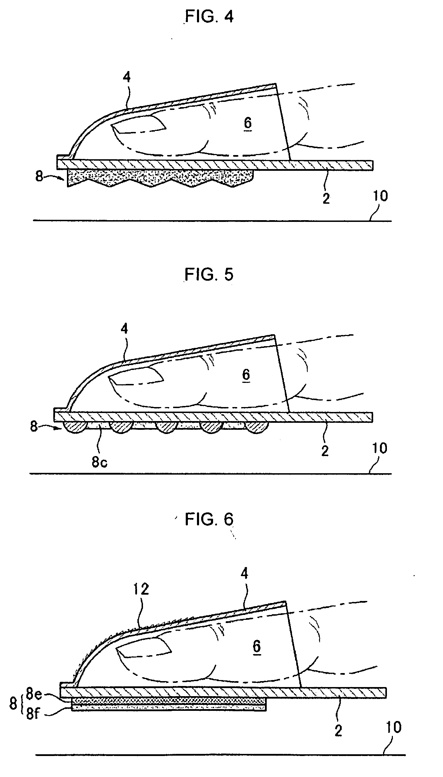

FIG. 4 shows a cross-sectional view of a tooth cleaner according to a fourth exemplary embodiment according to the present invention.

Differing from the above exemplary embodiments of the present invention, cotton or a sponge is used to form the tooth cleansing portion 8 according to the fourth exemplary embodiment of the present invention. The cotton or the sponge is put into the liquid mouth detergent for a predetermined time, and then it is dried.

FIG. 5 shows a cross-sectional view of a tooth cleaner according to a fifth exemplary embodiment of the present invention. According to the fifth exemplary embodiment of the present invention, soft silicon dots are formed on the substrate 2 in a predetermined pattern, the mouth detergent 8c is filled between the dots, and the protection film 10 covers an external surface of the tooth cleansing portion 8. The soft silicon is also referred to as a bioceramic.

In addition, the silicon dots may include the mouth detergent 8c. In this case, there is a merit in that a process for filling the mouth detergent 8c between the dots may be omitted.

The silicon dots forming the tooth cleansing portion 8 according to the fifth exemplary embodiment of the present invention may touch deeper in the gaps between the teeth so that the same effect as brushing the teeth with a tooth brush may be achieved.

FIG. 6 shows a cross-sectional view of a tooth cleaner according to a sixth exemplary embodiment of the present invention. A configuration of the tooth cleaner according to the sixth exemplary embodiment of the present invention is the same as that of the above exemplary embodiments of the present invention except that the tooth cleansing portion 8 includes a liquid mouth detergent layer 8e and a solid mouth detergent layer 8f.

Further, a polishing layer 12 for eliminating tartar from the teeth may be additionally provided to the tooth cleaner according to the sixth exemplary embodiment of the present invention. In the sixth exemplary embodiment of the present invention, the polishing layer 12 is provided on an outer surface of the sheet 4 forming the finger covering portion 6. Plastic micro-balls are combined with a polyvinyl or polyethylene binder to coat the polishing layer 12, and the plastic micro-balls are a component included in a tooth paste to be used to polish the teeth.

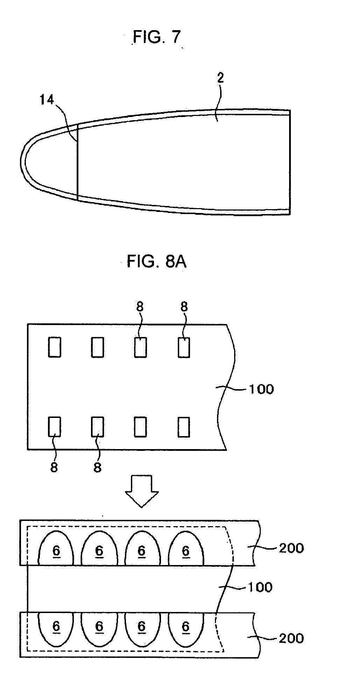

FIG. 7 shows a cross-sectional view of a tooth cleaner according to a seventh exemplary embodiment of the present invention. As shown in FIG. 7, dental floss 14 is provided crossing the substrate 2. The dental floss 14 is used to eliminate foreign materials from a tongue.

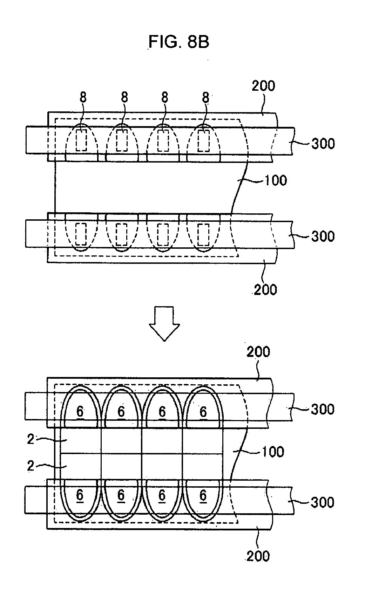

A manufacturing process of the tooth cleaner according to the exemplary embodiment of the present invention will now be described with reference to FIG. 8A and FIG. 8B. The tooth cleaner may be mass produced in the manufacturing process shown in FIG. 8A and FIG. 8B.

In FIG. 8A, ultrasonic bonding is performed to bond the tooth cleansing portion 8 on a surface of a material 100 for the substrate, and subsequently, a material 200 for the sheet is overlapped on both sides of another surface of the material 100. The ultrasonic bonding is performed to bond the material 100 to the material 200 by using an ultrasonic electrode (not shown) formed in a shape that is to the same as the finger covering portion 6, so that the finger covering portion 6 as shown in FIG. 6 is formed. The mouth detergent 8c may be previously provided to the tooth cleansing portion 8, or it may be forcibly provided to the tooth cleansing portion 8 by using an appropriate device after the tooth cleansing portion 8 is bonded to a predetermined area of the material 100.

Subsequently, as shown in FIG. 8B, a protection film material 300 is overlapped and bonded on the surface to which the tooth cleansing portion 8 is bonded, and the materials 100, 200, and 300 are passed through a mold to be formed in a tooth cleaner shape. Accordingly, the tooth cleaner may be sequentially produced, and mass production may be achieved.

Since a substrate is bonded on a sheet to form a finger covering portion and ultrasonic bonding is performed to bond a tooth cleansing portion on the substrate, it is appropriate for the mass production. In addition, a tooth cleansing effect is substantial since a water expansile material, a porous non-woven fabric, cotton, and a sponge provided as the tooth cleansing portion are appropriately touched on the teeth, and it is possible to elaborately cleanse and massage the teeth since the tooth cleansing portion is well touched on the gaps between the teeth.

In addition, since the silicon dots provided as the tooth cleansing portion may cleanse the gaps between the teeth, a tooth cleansing effect is further improved.

The above-described tooth cleansing portions include a cushion function, and therefore a massage function on the gingivae, the roof of the mouth, and the tongue may be performed. Particularly, when a user has a dental problem, the user may massage the teeth while controlling the strength used for brushing the teeth. Further, the tartar may be eliminated by using the polishing layer, and foreign materials on the tongue may be eliminated by using the dental floss.

The mouth detergent in the tooth cleansing portion may be selectively provided for a supplement for gum clinic, or for cleansing the mouth, and it may be used for a pet dog.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

## Claims

1. A tooth cleaner comprising a substrate, a sheet attached to one surface of the substrate to form a finger cover, and a tooth cleansing portion attached to another surface of the substrate, the tooth cleansing portion having a mouth detergent, wherein the tooth cleansing portion comprises a paste having the mouth detergent and covering the other surface of the substrate.

2. The tooth cleaner of claim 1, wherein the tooth cleansing portion comprises cotton or a sponge having the mouth detergent.

3. The tooth cleaner of claim 1, wherein the tooth cleansing portion comprises two embossed paper napkins that are symmetrically superposed, and a space formed between the two embossed paper napkins that are symmetrically superposed is charged with the mouth detergent.

4. The tooth cleaner of claim 1, wherein the tooth cleansing portion comprises a water expansile material having the mouth detergent.

5. The tooth cleaner of claim 1, wherein the tooth cleansing portion comprises a porous non-woven fabric charged with the mouth detergent.

6. The tooth cleaner of claim 1, wherein the tooth cleansing portion comprises soft silicon dots arranged in a predetermined pattern, and the mouth detergent is charged between the soft silicon dots.

7. The tooth cleaner of claim 1, wherein the tooth cleansing portion comprises silicon dots formed by the mouth detergent and arranged in a predetermined pattern.

8. The tooth cleaner of claim 1, wherein the tooth cleansing portion comprises a liquid mouth detergent layer and a solid mouth detergent layer, and the liquid and solid mouth detergent layers are stacked on the other surface of the substrate.

9. The tooth cleaner of claim 1, further comprising a polishing layer provided on an outer surface of the sheet to eliminate tartar from teeth.

10. The tooth cleaner of claim 1, further comprising dental floss for cleansing a tongue and provided on the other surface of the substrate such that the dental floss is not overlapped with the tooth cleansing portion.
